(19) Europäisches Patentamt European Patent Office Office européen des brevets

(11) **EP 4 737 892 A1**

(12) **EUROPEAN PATENT APPLICATION**
published in accordance with Art. 153(4) EPC

(43) Date of publication:
06.05.2026 Bulletin 2026/19

(21) Application number: 24845098.3

(22) Date of filing: 15.03.2024

(51) International Patent Classification (IPC):
*G01N 27/02* (2006.01)

(52) Cooperative Patent Classification (CPC):
G01N 27/02

(86) International application number:
PCT/JP2024/010219

(87) International publication number:
WO 2025/022713 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.07.2023 JP 2023122104

(71) Applicant: JFE Steel Corporation
Tokyo, 100-0011 (JP)

(72) Inventor: KIYOIZUMI, Kota
Tokyo 100-0011 (JP)

(74) Representative: Haseltine Lake Kempner LLP
Bürkleinstrasse 10
80538 München (DE)

(54) **METHOD FOR ANALYZING MOLTEN SLAG**

(57) The present invention provides a molten slag analysis method that can quickly and accurately obtain the basicity and the iron oxide content of molten slag based on a relationship between the impedance between electrodes immersed in the molten slag and the temperature of the molten slag. This method is characterized by including: a first step of measuring a reference slag temperature of reference molten slag, and measuring a reference impedance of the reference molten slag by applying an alternating current between a pair of electrodes immersed in the reference molten slag at the reference slag temperature; a second step of creating a constituent content calibration curve from a content of the constituent included in the reference molten slag and the reference impedance measured in the first step; a third step of measuring a slag temperature of molten-slag-being-measured, and measuring a measured impedance at the slag temperature; and a fourth step of estimating a content of the constituent included in the molten-slag-being-measured based on the measured impedance and the constituent content calibration curve.

[Fig. 3]

(a)

R² = 0.9444

Estimated Basicity (-) / Actual Basicity (-)

(b)

R² = 0.9476

Estimated Basicity (-) / Actual Basicity (-)

EP 4 737 892 A1

**Description**

Technical Field

The present invention relates to a molten slag analysis method. In particular, the present invention relates to a molten slag analysis method that can obtain the basicity and the iron oxide content of molten slag based on a relationship between an impedance between a pair of electrodes immersed in the molten slag at a predetermined temperature of the molten slag and the content of a constituent included in the molten slag.

Background Art

In a steel refining process, the main purpose of a molten steel refining step is to remove impurities included in the molten steel, such as phosphorus, silicon, and sulfur, by supplying a refining agent, such as a CaO source, or oxygen to molten iron that is the main raw material of the molten steel. Generally, refining of molten steel is performed by oxidation refining or reduction refining. In steel refining, therefore, the basicity is employed as an index to evaluate the intensity of the basicity of molten slag. This basicity can be represented, for example, by a ratio between the content of CaO (%CaO) and the content of $SiO_2$ (%$SiO_2$) included in the molten slag. Thus, the basicity can be represented by (%CaO)/(%$SiO_2$).

In the molten steel refining step, feeding a predetermined amount of refining agent, such as a CaO source, into the molten steel can increase the basicity of the molten slag and thereby promote an oxidation reaction of the molten steel. In the molten steel refining step, a dephosphorization reaction of the molten steel is accompanied by a reduction reaction of iron oxide included in molten slag. Therefore, by manipulating the oxygen feeding speed and the level of a lance through which oxygen is supplied, the content of iron oxide included in the molten steel can be adjusted so as to promote the dephosphorization reaction of the molten steel.

Thus, for impurities included in the molten steel, such as phosphorus, silicon, and sulfur, it is necessary to determine the amount to be fed of a refining agent, such as a CaO source, according to the amount to be removed of target impurity components and to feed the predetermined amount of refining agent into the molten steel. In the molten steel refining step, therefore, it is extremely important to analyze the slag composition of the molten slag present inside a furnace.

To analyze the slag composition of molten slag, a quantitative analysis using the intensity of a fluorescent X-ray (hereinafter referred to as "fluorescent X-ray analysis method") has been hitherto widely adopted. A slag composition quantitative analysis method for molten slag using this fluorescent X-ray analysis method is as follows: First, part of the molten slag present inside a furnace is sampled, and the sampled slag is transferred to an analysis room to prepare a specimen for analysis. Thereafter, the prepared specimen for analysis is irradiated with a fluorescent X-ray (primary X-ray). Then, the intensity of a fluorescent X-ray (secondary X-ray) that is emitted from the specimen for analysis as the specimen for analysis is irradiated with the fluorescent X-ray (primary X-ray) is measured for each element. Finally, the content of each element is obtained from the measured value of the intensity of the fluorescent X-ray using a calibration curve indicating a relationship between the intensity of the fluorescent X-ray of each element and the content of each element that is created beforehand.

In the fluorescent X-ray analysis method, however, determining the amount of each constituent included in molten slag with high accuracy requires preparing a specimen for analysis. In the fluorescent X-ray analysis method, preparing a specimen for analysis needed to determine the amount of each constituent included in molten slag with high accuracy requires a time of at least ten minutes or longer.

Meanwhile, the slag composition of molten slag during converter blowing changes incessantly with time according to the degree of progress of the blowing. It is therefore grossly inaccurate to determine the amount to be fed of a refining agent or the blowing pattern based on the analysis result of the slag composition of the molten slag obtained by implementing the fluorescent X-ray analysis method.

Under these circumstances, quick analysis technologies for slag compositions have been proposed. For example, Patent Literature 1 proposes a slag constituent analysis method that can quickly and highly accurately measure a slag constituent generated in refining molten iron. Specifically, the slag constituent analysis method described in Patent Literature 1 is an analysis method that irradiates a surface of a slag-being-analyzed with a pulsed laser multiple times to turn part of the slag into a plasma, and derives a target constituent concentration or constituent amount ratio from an emission spectrum that is acquired by spectrally dispersing excitation light obtained from the slag turned into a plasma.

Further, Patent Literature 2 proposes an impedance and phase angle difference measurement probe that is used to accurately control the composition of a slag, and a slag composition control method using this probe. Specifically, the slag composition control method described in Patent Literature 2 is a method in which a measurement probe including at least three electrodes is immersed into the molten slag and an impedance measurement is performed while the combination of the electrodes is changed to thereby separately calculate a charge transfer resistance and a solution resistance, and the basicity of the slag and the content of iron oxide (%FeO) included in the molten slag are obtained using calibration curves created beforehand.

Citation List

Patent Literature

Patent Literature 1: Japanese Patent No. 7111282
Patent Literature 2: JP-A-2022-110303

Summary of Invention

Technical problem

However, the conventional technologies have the following problems yet to be solved: the slag constituent analysis method described in Patent Literature 1, which requires, due to the influence of heat and dust, performing a measurement of an emission spectrum acquired by spectrally dispersing excitation light that is obtained from a slag turned into a plasma at a sufficiently distant position during slag removal after completion of blowing. Therefore, the slag constituent analysis method described in Patent Literature 1 cannot be applied during blowing during which the influence of heat and dust is significant.

While the slag composition control method described in Patent Literature 2 is a technology that can analyze a slag composition during blowing, this method has some problems. First, the slag composition control method described in Patent Literature 2 has difficulty in quickly analyzing the slag composition of molten slag. Due to the characteristics of the impedance measurement, this slag composition control method requires measuring a plurality of impedances with different frequencies within a set range of frequency, and obtaining a resistance value of each element in an equivalent circuit by performing fitting. Thus, the slag composition control method described in Patent Literature 2 takes a lot of time to obtain the resistance value of each element in the equivalent circuit.

Further, the slag composition control method described in Patent Literature 2 requires performing the impedance measurement of molten slag in at least three patterns with the combination of the electrodes changed. Thus, in a situation where the slag properties of molten slag are likely to change during operation of a converter, the slag composition control method described in Patent Literature 2 has difficulty in maintaining its accuracy regarding the content of a slag constituent included in the molten slag.

Moreover, in the slag composition control method described in Patent Literature 2, the solution resistance and the charge transfer resistance obtained by the impedance measurement of the molten slag fluctuate significantly according to a change in the temperature of the molten slag. Therefore, as the calibration curves that are used in the slag composition control method described in Patent Literature 2 and that need to be created and prepared beforehand, calibration curves corresponding to the slag temperature of the molten slag at the time of the impedance measurement should be used. However, the calibration curves used in the slag composition control method described in Patent Literature 2 do not correspond to the slag temperature of the molten slag.

Having been developed in view of these circumstances, the present invention aims to provide a molten slag analysis method that can quickly and accurately obtain the basicity and the iron oxide content of molten slag based on a relationship between an impedance between electrodes immersed in the molten slag and the temperature of the molten slag.

Solution to Problem

To solve the above problems, the present inventor conducted various experiments, which resulted in gaining an insight that the content of a constituent included in molten slag could be accurately and quickly obtained based on a relationship between an impedance between a pair of electrodes immersed in the molten slag, which corresponds to the slag temperature of the molten slag, and the content of the constituent included in the molten slag. The present invention has been developed based on this insight, and is summarized as follows.

That is, a molten slag analysis method according to the present invention that advantageously solves the above-described problems is a method that analyzes a constituent of molten slag and is characterized by including: a first step of measuring a reference slag temperature of reference molten slag, and measuring a reference impedance of the reference molten slag by applying an alternating current between a pair of electrodes immersed in the reference molten slag at the reference slag temperature; a second step of creating a constituent content calibration curve from a content of the constituent included in the reference molten slag and the reference impedance measured in the first step; a third step of measuring a slag temperature of molten-slag-being-measured, and measuring a measured impedance by applying the alternating current between the pair of electrodes immersed in the molten-slag-being-measured at the slag temperature; and a fourth step of estimating a content of the constituent included in the molten-slag-being-measured based on the measured impedance and the constituent content calibration curve.

In the molten slag analysis method according to the present invention, the following are preferable solutions: (a) that the constituent is iron oxide, and that the constituent content calibration curve is an iron oxide content calibration curve; (b) that the constituent is a specific constituent needed to calculate a basicity of the reference molten slag, and that the constituent content calibration curve is a basicity calibration curve; (c) that the reference impedance and the measured impedance are measured by applying alternating currents of two selected types of frequencies; (d) that a voltage that is applied between the pair of electrodes is 10 mV or higher but 1.0 V or lower, and that the two types of frequencies are respectively 10 Hz or lower and 10 kHz or higher; and (e) that a difference in immersion potentials in the molten-slag-being-measured between the pair of electrodes is less than 50 mV.

Advantageous Effects of Invention

The present invention can quickly and accurately estimate the basicity and the iron oxide content of molten slag without sampling the molten slag during blowing.

Brief Description of Drawings

[Fig. 1] is a schematic view showing an overview of a molten slag analysis device that is used to implement a molten slag analysis method according to an embodiment.

[Fig. 2] is a graph showing behavior of the impedance of molten slag when a frequency is swept using the molten slag analysis device that is used to implement the molten slag analysis method according to the embodiment.

[Fig. 3] is an estimation result of a basicity on a calibration curve obtained by performing a multiple regression analysis based on a solution resistance and a charge transfer resistance of a reference slag in an example, with Fig. 3 (a) being an estimation result of the basicity at 1290 to 1310°C, and Fig. 3 (b) being an estimation result of the basicity at 1390 to 1410°C.

[Fig. 4] is an estimation result of an iron oxide content on a calibration curve obtained by performing a multiple regression analysis based on the solution resistance and the charge transfer resistance of the reference slag in an example, with Fig. 4 (a) being an estimation result of the iron oxide content at 1290 to 13 10°C, and Fig. 4 (b) being an estimation result of the iron oxide content at 1390 to 1410°C.

Description of Embodiments

[First Embodiment]

A molten slag analysis method according to a first embodiment will be described. The molten slag analysis method according to this embodiment includes: a first step of measuring a reference slag temperature of reference molten slag, and measuring a reference impedance of the reference molten slag by applying an alternating current between a pair of electrodes immersed in the reference molten slag at the reference slag temperature; a second step of creating a constituent content calibration curve from a content of the constituent included in the reference molten slag and the reference impedance measured in the first step; a third step of measuring a slag temperature of molten-slag-being-measured, and measuring a measured impedance by applying the alternating current between the pair of electrodes immersed in the molten-slag-being-measured at the slag temperature; and a fourth step of estimating a content of the constituent included in the molten-slag-being-measured based on the measured impedance and the constituent content calibration curve.

Thus, the molten slag analysis method according to this embodiment creates a constituent content calibration curve from the content of the constituent included in the reference molten slag and the measured reference impedance, and can estimate the content of the constituent included in the molten-slag-being-measured based on the measured impedance of the molten-slag-being-measured and the constituent content calibration curve.

In the following, a molten slag analysis device that is used for the molten slag analysis method according to this embodiment will be described.

<Overview of Molten Slag Analysis Device>

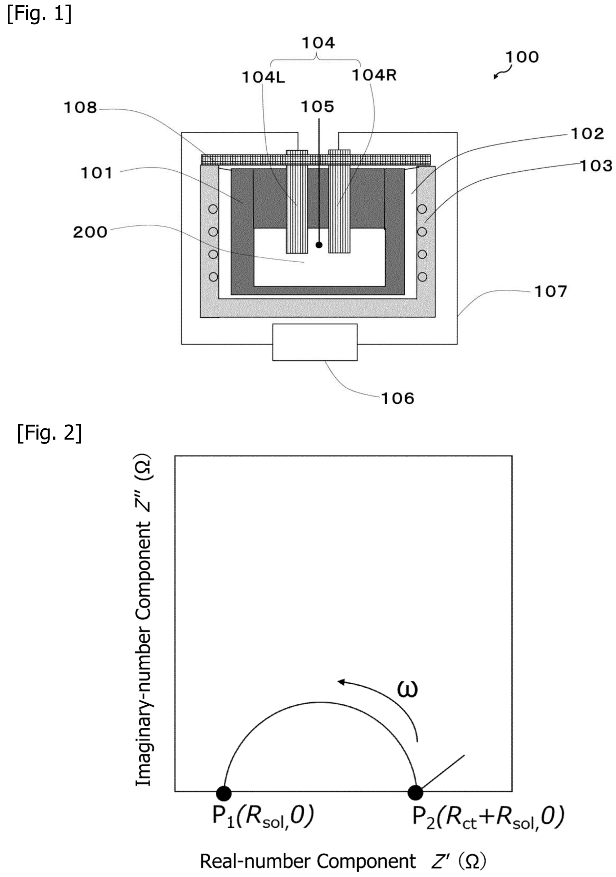

Fig. 1 is a schematic view showing an overview of the molten slag analysis device that is used for the molten slag analysis method according to this embodiment. As shown in Fig. 1, a molten slag analysis device 100 includes a crucible 101, a ramming material 102, and an induction melting furnace 103. In the crucible 101, molten iron that is formed by melting scrap, molten pig iron, etc. is charged. It is preferable that the crucible 101 be made of a material that has low solubility for molten iron and is thermodynamically stable.

As the crucible 101, an MgO crucible, a C crucible, a CaO crucible, or an $Al_2O_3$ crucible can be used. While the shape of the crucible 101 is not particularly restricted, it may be a columnar shape. When the shape of the crucible 101 is a columnar shape, the cross-sectional area thereof may be 0.005 to 0.030 m$^2$ and may be, for example 0.018 m$^2$.

The ramming material 102 is a refractory. The ramming material 102 covers a side wall and a bottom surface of the crucible 101 and is attached to an inner wall of the induction melting furnace 103. It is preferable that the thickness of the ramming material 102 be 100 to 150 mm from the viewpoint of heat resistance and durability. The induction melting furnace 103 is a crucible-type low-frequency induction furnace. The induction melting furnace 103 can hold molten iron in a state of maintaining a high temperature. An induction heater may be provided on a side wall of the induction melting furnace 103.

Further, the molten slag analysis device 100 includes a pair of electrodes 104 for applying an alternating current to molten slag 200 charged in the crucible 101. The pair of electrodes 104 is provided to measure the impedance of the molten slag 200. Upper ends of the pair of electrodes 104 penetrate the crucible 101 and a heat insulation board 108 and protrude to an outside of the molten slag analysis device 100. Lower ends of the electrodes 104 are immersed in the molten slag 200 present inside the crucible 101 to measure the impedance of the molten slag 200.

While it is preferable that the electrodes 104 be platinum electrodes, instead of platinum electrodes, electrodes made of tungsten, a zirconia-based refractory, a magnesia-based refractory, etc. that are chemically stable materials with low overvoltage can also be used. The form of the electrodes 104 may be a rod shape or a flat plate shape.

In the molten slag analysis device 100, the pair of electrodes 104 is formed by an electrode 104L installed on a left side and an electrode 104R installed on a right side. It is preferable that an inter-electrode distance formed between the electrode 104L and the electrode 104R be 5 to 500 mm. This inter-electrode distance can be changed as appropriate according to analysis conditions of the molten slag 200 adopted by the molten slag analysis method of this embodiment.

Further, the electrode 104L and the electrode 104R include a temperature sensor 105 for measuring the slag temperature of the molten slag 200. It is preferable that the electrodes 104 include the temperature sensor 105, because then the slag temperature of the molten slag 200 and the impedance of the molten slag 200 associated with that slag temperature can be measured. The temperature sensor 105 may be disposed between the electrode 104L and the electrode 104R. That is, if the temperature sensor 105 is installed near a wall surface of the crucible 101 instead of near the electrode 104L and the electrode 104R, there is a concern that deviation from an impedance measurement temperature may occur. Therefore, it is preferable that the temperature sensor 105 be installed near the electrodes of the electrode 104L and the electrode 104R that perform the impedance measurement. The electrodes 104 and the temperature sensor 105 may be configured as a probe mounted on a base material, and this probe may be mounted on a converter sublance.

Here, the slag temperature of the molten slag 200 is an important index in determining the amount to be added of a refining agent, such as a CaO source, that is fed into molten steel to control the basicity of the molten slag 200.

The electrode 104L mounted on the induction melting furnace 103 and a negative electrode of a potentiostat 106 installed outside the induction melting furnace 103 are connected to each other through a conductive wire 107. The electrode 104R mounted on the induction melting furnace 103 and a positive electrode of the potentiostat 106 installed outside the induction melting furnace 103 are connected to each other through a conductive wire 107. Thus, as both electrodes that are the electrode 104R and the electrode 104L forming a pair of electrodes are connected to the potentiostat 106 installed outside the induction melting furnace 103, an electric circuit serving as an equivalent circuit is formed.

Upper surfaces of the MgO crucible 101, the ramming material 102, and the induction melting furnace 103 included in the molten slag analysis device 100 are covered with the heat insulation board 108. The heat insulation board 108 functions to cover the upper surfaces of the crucible 101, the ramming material 102, and the induction melting furnace 103 and maintain the slag temperature of the molten slag 200 charged in the crucible 101. The heat insulation board 108 is not particularly restricted as long as the heat insulation board 108 may be any material that has heat insulation properties.

Next, each step included in the molten slag analysis method according to this embodiment that uses such a molten slag analysis device 100 will be described.

<First Step: Step of Measuring Slag Temperature and Impedance of Reference Molten Slag>

The molten slag analysis method according to this embodiment includes the first step of measuring the reference slag temperature of the reference molten slag, and measuring the reference impedance of the reference molten slag by applying an alternating current between the pair of electrodes immersed in the reference molten slag at the reference slag temperature.

Specifically, the first step is a step of measuring a reference slag temperature $T_{ref}$ and a reference impedance $Z_{ref}$ of the reference molten slag. The reference impedance $Z_{ref}$ of the reference molten slag is associated with the reference slag temperature $T_{ref}$. The reference impedance $Z_{ref}$ of the reference molten slag is measured to create a constituent content calibration curve indicating a relationship with the content of the constituent included in the reference molten slag.

In the first step included in the molten slag analysis method according to this embodiment, the reference impedance $Z_{ref}$ of the reference molten slag is measured by applying an alternating current between the pair of electrodes 104 immersed in the reference molten slag. The space between the pair of electrodes 104 immersed in the reference molten slag functions as an electrode for measuring the reference impedance $Z_{ref}$.

Here, the reference molten slag refers to molten slag 200 that is known molten slag for which the content of a constituent included in the reference molten slag has been already determined. That is, the reference molten slag means molten slag 200 that is used to create a constituent content calibration curve indicating a relationship between the content of the constituent included in the reference molten slag and the reference impedance $Z_{ref}$, and is a different molten slag 200 from the molten-slag-being-measured that is a measurement target in the third step to be described later.

Using the molten slag analysis device 100 shown in Fig. 1, a $CaO\text{-}SiO_2\text{-}FeO$-based molten slag 200 is formed as the reference molten slag inside, for example, a columnar C crucible as the crucible 101. Specifically, the ramming material 102 is embedded in an outer wall of the C crucible, and thereafter industrial pure iron is heated and melted into molten iron using the induction melting furnace 103. The molten iron may be manufactured with adjustments made such that the phosphorus concentration of phosphorus included in the molten iron falls within a predetermined range.

The molten slag 200 that can be applied to the molten slag analysis method according to this embodiment is not limited to a $CaO\text{-}SiO_2\text{-}FeO$-based molten slag, and the molten slag 200 may instead be a $CaO\text{-}SiO_2\text{-}MgO\text{-}FeO$-based, $CaO\text{-}SiO_2\text{-}Al_2O_3\text{-}FeO$-based, or $CaO\text{-}SiO_2\text{-}MgO\text{-}Al_2O_3\text{-}FeO$-based molten slag.

Further, in the system of the molten slag analysis device 100, flux is fed onto an upper surface of the molten iron to form reference molten slag as the molten slag 200 on the upper surface of the molten iron. The amount to be fed of flux can be determined as appropriate according to the internal volume of the crucible 101, the total amount of the molten iron, etc. The amount to be fed of the flux may be a ratio of, for example, 10 to 30 kg per ton of molten iron, preferably 15 to 25 kg per ton of molten iron, and more preferably 20 kg per ton of molten iron.

The constituents of the flux are not particularly restricted as long as a constituent that can form reference molten slag is included. The constituents of the flux may include, for example, $CaO$, $SiO_2$, $FeO$, $MgO$, etc.

When the flux includes $CaO$, $SiO_2$, $FeO$, and $MgO$ as its constituents, the contents thereof may be, for example, 22.5 (%CaO), 28.0 (%$SiO_2$), 42.5 (%FeO), and 7.0 (%MgO) on a mass basis.

After the flux is fed onto the upper surface of the molten iron, the molten steel temperature of the molten iron present inside the crucible 101 is maintained within a range of 1200 to 1700°C. As the flux is fed onto the molten iron and the molten steel temperature is maintained, reference molten slag and molten iron are formed in the system of the molten slag analysis device 100. Thereafter, an opening of the induction melting furnace 103 is closed with the heat insulation board 108 and the inside of the induction melting furnace 103 is maintained within a range of 1300 to 1600°C, preferably 1250 to 1500°C, and more preferably 1290 to 1310°C or 1390 to 1410°C.

The pair of electrodes 104 is immersed into the reference molten slag formed in the system of the molten slag analysis device 100. In the molten slag analysis device 100, the electrode 104L installed on the left side and the electrode 104R installed on the right side form the pair of electrodes 104. It is preferable that the electrode 104L and the electrode 104R have the same electrochemical properties.

Accordingly, the electrode 104L and the electrode 104R may be the same, or may be different if their electrochemical properties are the same. The inter-electrode distance formed by the two electrodes consisting of the electrode 104L and the electrode 104R is not particularly restricted, as long as the inter-electrode distance is such that the two electrodes 104 do not contact each other and the reference impedance of the reference molten slag can be accurately measured. The inter-electrode distance may be 5 to 50 mm or preferably 10 mm.

In the first step, the temperature sensor 105 first measures the reference slag temperature $T_{ref}$ of the reference molten slag. The reference impedance $Z_{ref}$ of the reference molten slag is measured by applying an alternating current between both electrodes that are immersed in the reference molten slag and are the pair of electrodes 104 formed by the electrode 104R and the electrode 104L.

Next, the reference impedance of the reference molten slag is measured by applying an alternating current between the pair of electrodes 104 immersed in the reference molten slag at the reference slag temperature $T_{ref}$. The current value of the alternating current applied between the pair of electrodes 104 is determined by the content of CaO (%CaO), the content of $SiO_2$ (%$SiO_2$), and the content of iron oxide (%FeO) included in the target slag, the concentration of impurities included therein, such as phosphorus, silicon, and sulfur, and the physical properties of the slag. Therefore, the current value is determined at the time point when a voltage to be applied between the pair of electrodes 104 is set.

The applied voltage to be applied between the pair of electrodes 104 is not particularly restricted, as long as the applied voltage allows the reference impedance $Z_{ref}$ to be accurately measured taking into account the content of CaO (%CaO), the content of $SiO_2$ (%$SiO_2$), and the content of iron oxide (%FeO) included in the reference molten slag and the concentration of impurities included therein, such as phosphorus, silicon, and sulfur.

The applied voltage to be applied between the pair of electrodes 104 may be 5.0 mV to 5.0 V or lower. The applied voltage to be applied between the pair of electrodes 104 being 5.0 mV or higher is preferable as this minimizes the influence of noise on the frequency response. The applied voltage to be applied between the pair of electrodes 104 being 5.0 V or lower is preferable because then the states of the electrode surfaces of the pair of electrodes 104 can be maintained.

Frequencies that are used to apply the applied voltage between the pair of electrodes 104 should be two types of frequencies. The two types of frequencies are set respectively to a high frequency side and a low frequency side.

The frequency on the high frequency side constituting one of the two types of frequencies is set to calculate a solution resistance $R_{sol}$ that is one component of the reference impedance $Z_{ref}$. The frequency on the low frequency side constituting the other of the two types of frequencies is set to calculate a charge transfer resistance $R_{ct}$ that is the other component of the reference impedance $Z_{ref}$. The frequency difference between the frequency on the high frequency side and the frequency on the low frequency side constituting the two types of frequencies is not particularly restricted, as long as these frequencies are two types of frequencies different from each other.

The frequencies that are used to apply the applied voltage between the pair of electrodes 104 may be generated by a variable signal generator (not shown) that is a sweep signal generator provided in the potentiostat 106.

In the first step included in the molten slag analysis method according to this embodiment, the measurement of the reference slag temperature $T_{ref}$ of the reference molten slag can be performed so as to cover an entire range expected for the reference slag temperature $T_{ref}$ of the reference molten slag during blowing in a converter. For example, the possible temperature range of the slag temperature of the reference molten slag may be set to 1200 to 1500°C, and a plurality of reference slag temperatures $T_{ref}$ ($T_{ref1}$, $T_{ref2}$, $T_{ref3}$, ... $T_{refn}$) of the reference molten slag may be measured in 5 to 10°C increments.

In the first step, the measurement of the reference impedance $Z_{ref}$ of the reference molten slag can be performed so as to cover all temperatures falling within a possible range of temperatures of the reference slag temperatures $T_{ref}$ ($T_{ref1}$, $T_{ref2}$, $T_{ref3}$, ... $T_{refn}$) of the reference molten slag. In the first step included in the molten slag analysis method according to this embodiment, also for each of reference molten slags in which the types of constituents included in the reference molten slag and the contents of the constituents are changed, the reference slag temperature can be measured so as to cover an entire range of temperatures corresponding to a possible range of temperatures of the reference slag temperatures $T_{ref}$ ($T_{ref1}$, $T_{ref2}$, $T_{ref3}$, ... $T_{refn}$) of the reference molten slag, and the reference impedance $Z_{ref}$ ($Z_{ref1}$, $Z_{ref2}$, $Z_{ref3}$, ... $Z_{refn}$) corresponding to that reference slag can be measured.

Thus, in the first step, the measurement of the reference slag temperature $T_{ref}$ of the reference molten slag and the measurement of the reference impedance $Z_{ref}$ thereof are performed so as to cover the entire range expected for the reference slag temperature $T_{ref}$ of the reference molten slag during blowing in the converter, which makes it possible to accurately obtain the relationship between the reference slag temperature $T_{ref}$ and the reference impedance $Z_{ref}$ of the reference molten slag.

<Second Step: Step of Creating Constituent Content Calibration Curve Based on Reference Impedance>

The molten slag analysis method according to this embodiment includes the second step of creating a constituent content calibration curve based on the content of the constituent included in the reference molten slag and the reference impedance measured in the first step. In the second step, a combined resistance of the reference molten slag is calculated based on the reference impedance $Z_{ref}$ of the reference molten slag measured at the pair of electrodes 104 formed by the electrode 104R and the electrode 104L.

Here, the combined resistance of the reference molten slag refers to the sum of the solution resistance $R_{sol}$ and the charge transfer resistance $R_{ct}$ of the reference molten slag. From the value of the combined resistance of the reference molten slag, the solution resistance $R_{sol}$ of the reference molten slag and the charge transfer resistance $R_{ct}$ of the reference molten slag can be each separately calculated.

From the value of the combined resistance of the reference molten slag, the solution resistance $R_{sol}$ and the charge transfer resistance $R_{ct}$ of the reference molten slag are calculated as follows.

Normally, when performing an impedance measurement of molten slag, a large number of measurement points are set to obtain an impedance measurement curve, with the frequency varied little by little, and the combined resistance of the molten slag is measured. The impedance measurement result of the molten slag can be represented by a graph of which the horizontal axis shows a real-number component of the combined resistance of the molten slag and the vertical axis shows an imaginary-number component of the combined resistance of the molten slag. A locus indicating the impedance measurement result of the molten slag shown in the graph forms a semicircle. Two points are formed as points at which this semicircle touches the horizontal axis showing the real-number component of the combined resistance of the molten slag.

From one of these points at which the semicircle touches the horizontal axis, the solution resistance value $R_{sol}$ of

the reference molten slag can be calculated, and from the other one of these points, the sum of the solution resistance $R_{sol}$ and the charge transfer resistance $R_{ct}$ of the reference molten slag can be calculated. Thus, the points touching the horizontal axis respectively represent the solution resistance $R_{sol}$ of the reference molten slag and the solution resistance $R_{sol}$ + charge transfer resistance $R_{ct}$ of the reference molten slag.

From this technical viewpoint, it was found that in the molten slag analysis method according to this embodiment, if the resistance values at the two points at which the semicircle touched the horizontal axis showing the real-number component of the combined resistance of the molten slag were known, the solution resistance $R_{sol}$ + charge transfer resistance $R_{ct}$ could be calculated by subtraction.

Thus, the molten slag analysis method according to this embodiment does not require plots at intermediate positions in the semicircle, for example, and instead involves performing the impedance measurement of the molten slag at only the frequencies touching the horizontal axis. Accordingly, the measurement time of the solution resistance $R_{sol}$ of the reference molten slag and the solution resistance $R_{sol}$ + charge transfer resistance $R_{ct}$ of the reference molten slag can be reduced.

Thus, in the second step, the combined resistance of the reference molten slag can be separated into its components, namely the solution resistance $R_{sol}$ and the charge transfer resistance $R_{ct}$, and these components can be quickly calculated.

Specifically, calculating each of the solution resistance $R_{sol}$ and the charge transfer resistance $R_{ct}$ of the reference molten slag from the reference impedance $Z_{ref}$ of the reference molten slag and the value of the combined resistance of the reference molten slag requires creating a Nyquist plot of the reference molten slag. Then, the combined resistance of the reference molten slag is calculated from the created Nyquist plot of the reference molten slag and Relational Expression (1) below.

Here, a Nyquist plot is an impedance spectrum plotted on a complex plane, and is generally plotted so as to draw a semicircle with respect to a solid line. A Nyquist plot curve can be obtained by a plurality of Nyquist plots based on numerous pieces of experimental data for drawing a semicircle.

That is, obtaining a Nyquist plot curve usually takes time and effort to measure numerous reference impedances $Z_{ref}$ and combined resistances of the reference molten slag to form numerous Nyquist plots and then perform fitting using these numerous Nyquist plots.

[Expression 1]

$$Z = Rsol + \frac{2Rct}{1+j\omega R_{ct}C_{dl}} \qquad (1)$$

In this Relational Expression (1), Z represents the reference impedance of the reference molten slag; $R_{sol}$ represents the solution resistance; $R_{ct}$ represents the charge transfer resistance; j represents an imaginary-number unit; $\omega$ represents an angular frequency; and $C_{dl}$ represents a double-layer capacitance.

$R_{sol}$, $R_{ct}$, and $C_{dl}$ are impedance components obtained by an analysis of an electric circuit serving as an equivalent circuit formed by the molten slag analysis device 100.

Fig. 2 is a graph showing behavior of the impedance of the molten slag when the frequency is swept using the molten slag analysis device used for the molten slag analysis method according to this embodiment. In Fig. 2, the horizontal axis shows the real-number component of the impedance and the vertical axis shows the imaginary-number component of the impedance.

As shown in Fig. 2, in the Nyquist plot curve, of intersection points $P_1$, $P_2$ between the Nyquist plot curve and the horizontal axis, the value of the horizontal axis at the intersection point $P_2$ ($R_{sol}$+$R_{ct}$, 0) that lies farther from the origin (0, 0) corresponds to the combined resistance $R_{sol}$+$R_{ct}$ of the reference molten slag. On the other hand, of the intersection points $P_1$, $P_2$ between the Nyquist plot curve and the horizontal axis, the value of the horizontal axis at the intersection point $P_1$ ($R_{sol}$, 0) that lies closer to the origin (0, 0) corresponds to the solution resistance $R_{sol}$ of the reference molten slag.

Thus, separately calculating each of the solution resistance $R_{sol}$ and the charge transfer resistance $R_{ct}$ of the reference molten slag from the reference impedance $Z_{ref}$ of the reference molten slag and the value of the combined resistance of reference molten slag does not require extremely numerous Nyquist plots forming a Nyquist plot curve, and instead involves calculating only the values of the horizontal axis at the intersection points $P_1$, $P_2$ between the Nyquist plot curve and the horizontal axis.

That is, the two points at which the plots converge with the horizontal axis correspond to $R_{sol}$ on the high frequency side and $R_{sol}$+$R_{ct}$ on the low frequency side, and therefore being able to measure only each of the points of converging with the real-number axis suffices. The solution resistance $R_{sol}$ and the charge transfer resistance $R_{ct}$ can be obtained by measuring the impedances at only two frequencies, without executing numerous Nyquist plots for creating a Nyquist plot curve.

Thus, the molten slag analysis method according to this embodiment can quickly and accurately obtain the solution resistance $R_{sol}$ and the charge transfer resistance $R_{ct}$ by the impedance measurements at two frequencies.

Here, the reason why only one semicircle appears in the rough shape of the impedances shown in Fig. 2 is that electrodes made of the same material are used as the pair of electrodes 104 included in the molten slag analysis device 100 so as to be equivalent to each other in electrochemical properties, so that the semicircles lie on top of each other so as to be apparently one semicircle.

That is, in the molten slag analysis method according to this embodiment, thus selecting electrodes similar in electrochemical properties is intended to help interpret a Nyquist diagram.

The solution resistance $R_{sol}$ of the reference molten slag is the liquid resistance of the molten slag between the pair of electrodes 104, and depends on the inter-electrode distance formed between the pair of electrodes 104. The charge transfer resistance $R_{ct}$ of the reference molten slag is a transfer resistance to charges that transfer according to an electrode reaction at the electrodes 104, and does not depend on the inter-electrode distance formed between the pair of electrodes 104.

In the second step, a constituent content calibration curve is created based on the solution resistance $R_{sol}$ and the charge transfer resistance $R_{ct}$ of the reference molten slag. The constituent content calibration curve can be created by a multiple regression analysis. Specifically, the constituent content calibration curve can be created from a regression expression obtained by multiple regression that uses, as the objective variable, the content of the constituent included in the reference molten slag that has been already determined or prepared and determined beforehand, and as the explanatory variable, the solution resistance $R_{sol}$ and the charge transfer resistance $R_{ct}$ of the reference molten slag.

The constituent content calibration curve may be intended for all of the constituents included in the reference molten slag and the constituents included in the molten-slag-being-measured, and may be for CaO, $SiO_2$, FeO, $Al_2O_3$, MgO, MnO, $P_2O_5$, silicon, sulfur, etc.

In the second step, the constituent included in the reference molten slag may be iron oxide, and the constituent content calibration curve may be an iron oxide content calibration curve. That is, in the second step, with a focus on iron oxide needed to remove phosphorus included in the molten pig iron by a dephosphorization reaction, an iron oxide content calibration curve is created as the constituent content calibration curve of the reference molten slag used for estimating the content of iron oxide.

Alternatively, in the second step, the constituent may be a specific constituent needed to calculate the basicity of the reference molten slag, and the constituent content calibration curve may be a basicity calibration curve. That is, in the second step, with a focus on the content of CaO and the content of $SiO_2$ for calculating the basicity that is a measure of the intensity of the basicity of the molten slag, a basicity calibration curve is created as the constituent content calibration curve of the reference molten slag used to calculate a predetermined basicity that is specified from these contents.

This basicity can be set as appropriate according to the constituents included in the reference molten slag. When the reference molten slag is a CaO-$SiO_2$-FeO-based molten slag, the basicity of the reference molten slag may be a basicity B1 defined by Relational Expression (2) below. This basicity can be calculated by dividing the mass percentage of CaO included in the reference molten slag by the mass percentage of $SiO_2$. The basicity B1 is the most important index in an actual converter. This means that the molten slag analysis method according to this embodiment has technical significance in that it can highly accurately estimate the basicity B1 that is the most important index in an actual converter.

[Expression 2]

$$\text{Basicity B1} = \frac{CaO}{SiO_2} \qquad (2)$$

In this Relational Expression (2), CaO represents the mass percentage of calcium oxide and $SiO_2$ represents the mass percentage of silicon dioxide.

The reference molten slag may be, for example, a CaO-$SiO_2$-MgO-$Al_2O_3$-FeO-based molten slag 200. The basicity of the reference molten slag may be a basicity B2 defined by Relational Expression (3) below. This basicity B2 can be calculated by dividing the sum of the mass percentage of CaO, the mass percentage of MgO, and the mass percentage of $Al_2O_3$ included in the reference molten slag by the mass percentage of $SiO_2$.

[Expression 3]

$$\text{Basicity B2} = \frac{(CaO + MgO + Al_2O_3)}{SiO_2} \qquad (3)$$

In this Relational Expression (3), CaO represents the mass percentage of calcium oxide; MgO represents the mass percentage of magnesium oxide; $Al_2O_3$ represents the mass percentage of aluminum oxide; and $SiO_2$ represents the mass percentage of silicon dioxide.

<Third Step: Step of Measuring Slag Temperature and Impedance of Molten-Slag-Being-Measured>

The molten slag analysis method according to this embodiment includes the third step of measuring the slag temperature of the molten-slag-being-measured, and measuring the measured impedance by applying an alternating current between the pair of electrodes immersed in the molten-slag-being-measured at the slag temperature.

That is, in the third step, for the molten-slag-being-measured, a slag temperature T and a measured impedance Z of the molten-slag-being-measured are measured in the same manner as in the first and second steps. Further, based on the measured impedance Z, a solution resistance $R_{sol}$ and a charge transfer resistance $R_{ct}$ of the molten-slag-being-measured are each separately calculated in the same manner as in the first and second steps. The space between the pair of electrodes 104 immersed in the molten-slag-being-measured functions as a measurement electrode for measuring the measured impedance Z.

<Fourth Step: Step of Estimating Constituent Content of Molten-Slag-Being-Measured Based on Calibration Curve>

The molten slag analysis method according to this embodiment includes the fourth step of estimating the content of the constituent included in the molten-slag-being-measured based on the measured impedance and the constituent content calibration curve. That is, the fourth step is a step of estimating, based on the constituent content calibration curve created in the second step, the content of the constituent corresponding to the solution resistance $R_{sol}$ and the charge transfer resistance $R_{ct}$ of the molten-slag-being-measured that are calculated from the measured impedance Z measured at the slag temperature T of the molten-slag-being-measured.

Here, when the constituent content calibration curve created in the second step is an iron oxide content calibration curve, the content of iron oxide included in the molten-slag-being-measured corresponding to the slag temperature T of the molten-slag-being-measured can be estimated. When the constituent content calibration curve created in the second step is a basicity calibration curve, the basicity of the molten-slag-being-measured can be estimated from the content of CaO and the content of $SiO_2$ included in the molten-slag-being-measured corresponding to the slag temperature T of the molten-slag-being-measured.

Based on the content of the iron oxide and the basicity of the molten-slag-being-measured that have been estimated in the fourth step, an appropriate amount of a refining agent to be fed into the molten steel can be determined. As an appropriate amount of refining agent is fed into the molten steel, an increase in the manufacturing cost due to feeding of an excessive amount of quicklime into the molten steel can be reduced. Further, as an appropriate amount of refining agent is fed into the molten steel, foaming at a late stage of dephosphorization blowing is inhibited, and failure of slag removal in the middle of the process and progress of a rephosphorization reaction during a decarburization blowing, etc. are avoided.

In addition, the blowing pattern can be determined based on the content of the iron oxide and the basicity of the molten-slag-being-measured that have been estimated in the fourth step.

Thus, by including the first step to the fourth step, the molten slag analysis method according to this embodiment can calculate the solution resistance $R_{sol}$ and the charge transfer resistance $R_{ct}$ based on the reference slag temperature $T_{ref}$ and the reference impedance $Z_{ref}$ of the reference molten slag without sampling the molten slag during blowing to create a constituent content calibration curve, and can quickly and accurately estimate the constituent content of the molten-slag-being-measured using that calibration curve.

As has been described above, the invention according to the first embodiment can quickly and accurately estimate the basicity and the iron oxide content of molten slag without sampling the molten slag during blowing.

[Second Embodiment]

A molten slag analysis method according to a second embodiment will be described. The molten slag analysis method according to this embodiment is characterized in that, in the molten slag analysis method according to the above-described embodiment, the voltage that is applied between the pair of electrodes is 10 mV or higher but 1.0 V or lower, and the two types of frequencies are respectively 10 Hz or lower and 10 kHz or higher.

In the following, characteristic parts included in the molten slag analysis method according to this embodiment will be described.

In the molten slag analysis method according to this embodiment, the voltage applied between the pair of electrodes 104 is 10 mV or higher and 1.0 V or lower. It is preferable that the voltage applied between the pair of electrodes 104 be 10 mV or higher, because then the influence of noise does not appear in frequency response when applying the applied voltage between the pair of electrodes. It is preferable that the voltage applied between the pair of electrodes be 1.0 V or lower, because then the surface states of the pair of electrodes do not change.

Further, in the molten slag analysis method according to this embodiment, the two types of frequencies when applying the applied voltage between the pair of electrodes are respectively 10 Hz or lower and 10 kHz or higher. That is, the two types of frequencies when applying the applied voltage between the pair of electrodes are set respectively to 10 Hz or lower as the frequency on the low frequency side and 10 kHz or higher as the frequency on the high frequency side. This is preferable because then the reference impedance $Z_{ref}$ and the measured impedance $Z$ can be accurately measured, and the solution resistance $R_{sol}$ and the charge transfer resistance $R_{ct}$ of the molten-slag-being-measured can be accurately obtained based on the measured impedance $Z_{ref}$.

In the molten slag analysis method according to this embodiment, as the two types of frequencies when applying the applied voltage between the pair of electrodes, a frequency on the low frequency side and a frequency on the high frequency side are set. The frequency on the low frequency side may be set to 0.1 Hz or higher but 10 Hz or lower. The frequency on the high frequency side may be set to 10 kHz or higher but 100 kHz or lower.

Thus, with a focus on the voltage applied between the pair of electrodes and the two types of frequencies when applying the applied voltage between these electrodes, the molten slag analysis method according to this embodiment can accurately measure the reference impedance $Z_{ref}$ and the measured impedance $Z$. Then, based on the reference impedance $Z_{ref}$ and the measured impedance $Z$, this method can accurately calculate the solution resistance $R_{sol}$ and the charge transfer resistance $R_{ct}$ of the reference molten slag and the measured impedance $Z$ to create a constituent content calibration curve, and can estimate the content of the constituent included in the molten-slag-being-measured using that calibration curve.

As has been described above, by setting the voltage and two types of frequencies applied between the pair of electrodes, the invention according to the second embodiment can accurately measure the reference impedance and the measured impedance and thereby accurately estimate the basicity and the iron oxide content of the molten slag.

[Third Embodiment]

A molten slag analysis method according to a third embodiment will be described. The molten slag analysis method according to this embodiment is characterized in that, in the molten slag analysis method according to the above-described embodiment, the difference in immersion potentials in the molten-slag-being-measured between the pair of electrodes is less than 50 mV. In the following, characteristic parts included in the molten slag analysis method according to this embodiment will be described.

In the molten slag analysis method according to this embodiment, the difference in immersion potentials in the molten-slag-being-measured is less than 50 mV. The difference in immersion potentials in the molten-slag-being-measured is a potential difference measured between the pair of electrodes 104.

If the difference in immersion potentials in the molten-slag-being-measured is less than 50 mV, the electrochemical properties of the electrode 104R and the electrochemical properties of the electrode 104L forming the pair of electrodes 104 can be considered to be substantially the same. That is, as the electrochemical properties of the electrode 104R and the electrochemical properties of the electrode 104L forming the pair of electrodes 104 are substantially the same, the electrode 104R and the electrode 104L constituting the pair of electrodes 104 can be considered to be electrodes made of the same material.

Since the pair of electrodes 104 is electrodes made of the same material, in the second step included in the molten slag analysis method according to this embodiment, there is only one Nyquist curve formed by a semicircle that is used to calculate the solution resistance $R_{sol}$ and the charge transfer resistance $R_{ct}$ of the reference molten slag calculated from the reference impedance $Z_{ref}$. This is preferable because consequently the Nyquist curve that is used to calculate the solution resistance $R_{sol}$ and the charge transfer resistance $R_{ct}$ of the reference molten slag becomes clear as well as the accuracy of an analysis of this Nyquist curve improves.

On the other hand, if the difference in immersion potentials in the molten-slag-being-measured is 50 mV or more, the electrochemical properties of the electrode 104R and the electrochemical properties of the electrode 104L forming the pair of electrodes 104 cannot be considered to be substantially the same. Thus, since the pair of electrodes 104 cannot be considered to be electrodes made of the same material, in the second step included in the molten slag analysis method according to this embodiment, there never be just one Nyquist curve formed by a semicircle that is used to calculate the solution resistance $R_{sol}$ and the charge transfer resistance $R_{ct}$ of the reference molten slag calculated from the reference impedance $Z_{ref}$. This is not preferable because consequently the Nyquist curve that is used to calculate the solution resistance $R_{sol}$ and the charge transfer resistance $R_{ct}$ of the reference molten slag becomes unclear as well as the accuracy of an analysis of this Nyquist curve does not improve.

Thus, in the molten slag analysis method according to this embodiment, the difference in immersion potentials in the molten-slag-being-measured is set to be less than 50 mV, and thereby the electrochemical properties of the electrode 104R and the electrode 104L forming the pair of electrodes 104 are set to be substantially the same. In the molten slag analysis method according to this embodiment, the accuracy of the constituent content calibration curve is improved as the accuracy of the analysis of the Nyquist curve used to calculate the solution resistance $R_{sol}$ and the charge transfer resistance $R_{ct}$ of the reference molten slag is improved.

As has been described above, the invention according to the third embodiment can extremely accurately estimate the basicity and the iron oxide content of the molten slag by improving the accuracy of the analysis of the Nyquist curve used to calculate the solution resistance $R_{sol}$ and the charge transfer resistance $R_{ct}$ of the reference molten slag.

[Other Embodiments]

While the invention of the present application has been described with reference to the embodiments, the invention of the present application is not limited to the above-described embodiments. Various changes understandable to those skilled in the art can be made to the configurations and details of the invention of the present application within the technical scope of the invention of the present application. A system or a device that combines, in one way or another, separate characteristics included in the respective embodiments is also included in the technical scope of the present invention.

Examples

In the following, effects of the present invention will be specifically described based on examples of implementation, but the present invention is not limited to these examples of implementation.

(Invention Example 1)

Using the molten slag composition analysis method according to the present invention, an analysis of the slag composition of molten slag present inside a furnace during converter blowing was conducted. Specifically, a probe including two platinum electrodes and a temperature sensor for measuring the temperature of the molten slag was installed on a sublance inside a furnace of an induction melting furnace including a magnesium-oxide crucible and a ramming material. The distance between the platinum electrodes was set to 10 mm.

Next, the sublance with the probe mounted thereon was lowered during converter blowing so as to immerse the platinum electrodes and the temperature sensor into the molten slag, and the temperature and the impedance of the molten slag were measured. The impedance measurement of the molten slag was conducted under the conditions of an applied voltage of 1.0 V and measurement cycles of 10 kHz and 1.0 Hz.

Here, a basicity calibration curve was prepared that indicated a relationship between the impedance of the molten slag that was obtained beforehand and the basicity of the molten slag that was calculated from the content of each constituent included in the molten slag. Based on the basicity calibration curve, the basicity of the molten slag calculated from each constituent included in the molten slag was calculated from the measured value of the measured impedance.

Further, an iron oxide calibration curve was prepared that indicated a relationship between the impedance of the molten slag that was obtained beforehand and the iron oxide content included in the molten slag that was calculated from the content of each constituent included in the molten slag. Based on the iron oxide calibration curve, the content of the iron oxide included in the molten slag was calculated from the measured value of the measured impedance.

(Comparative Example 1)

Using a fluorescent X-ray analysis method, an analysis of the slag composition of molten slag present inside a furnace during converter blowing was conducted. Specifically, sampling of the molten slag was performed at the same time as the impedance measurement of the molten slag that was conducted in Invention Example 1, and after the obtained sample was processed into a specimen for analysis, the slag composition of the molten slag was analyzed by the fluorescent X-ray analysis method.

In the fluorescent X-ray analysis method, the prepared specimen for analysis was irradiated with a fluorescent X-ray (primary X-ray). The intensity of a fluorescent X-ray (secondary X-ray) that was emitted from the specimen for analysis by irradiating this specimen for analysis with the fluorescent X-ray (primary X-ray) was measured for each element. Then, using a calibration curve indicating a relationship between the intensity of the fluorescent X-ray of each element and the content of each element that was created beforehand, the content of each element was obtained from the measured value of the intensity of the fluorescent X-ray. Based on the content of each element included in the molten slag that was calculated from the measured value of the intensity of the fluorescent X-ray, the basicity and the iron oxide content of the molten slag were obtained.

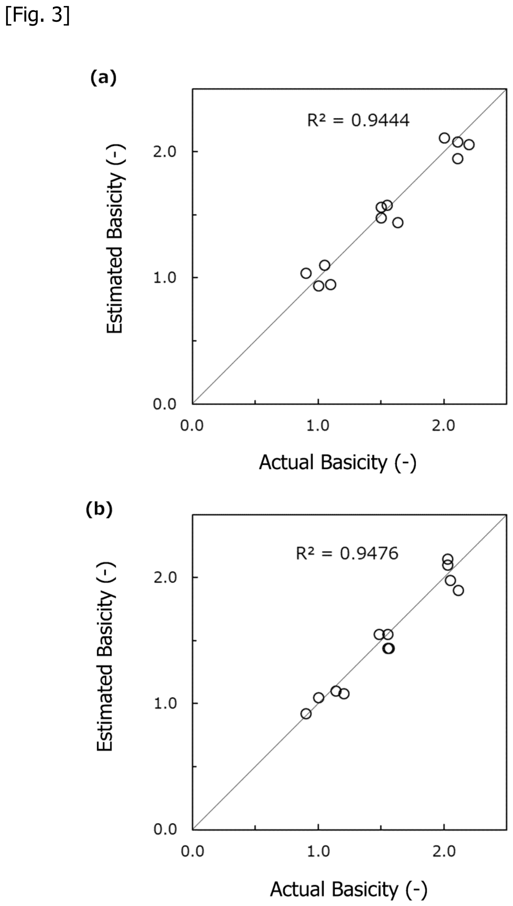

Fig. 3 shows the analysis result of the basicity of the molten slag measured in Invention Example 1 and Comparative Example 1. Specifically, Fig. 3 (a) is a graph showing by contrasting Invention Example 1 in which the basicity of the molten slag of which the temperature was 1290 to 1310°C was calculated using the molten slag composition analysis method according to the present invention with Comparative Example 1 in which the basicity was calculated using the fluorescent X-ray analysis method. Fig. 3 (b) is a graph showing by contrasting Invention Example 1 in which the basicity of the molten slag of which the temperature was 1390 to 1410°C was calculated using the molten slag analysis method according to the present invention with Comparative Example 1 in which the basicity was calculated using the fluorescent X-ray analysis method. (The result of the fluorescent X-ray analysis is expressed as "actual".)

Figs. 3 (a) and (b) revealed that regression coefficients $R^2$ calculated from these graphs were 0.9444 and 0.9476. These regression coefficients $R^2$ indicate that the molten slag composition analysis method according to the present invention can estimate the basicity of the molten slag with extremely high accuracy.

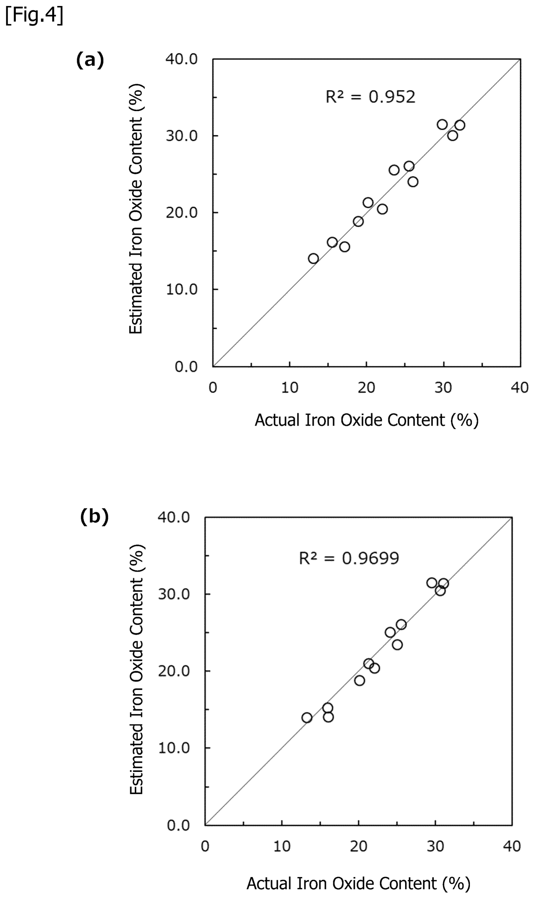

Fig. 4 shows the analysis result of the iron oxide content of the molten slag measured in Invention Example 1 and Comparative Example 1. Specifically, Fig. 4 (a) is a graph showing by contrasting Invention Example 1 in which the iron oxide content of the molten slag of which the temperature was 1290 to 13 10°C was calculated using the molten slag composition analysis method according to the present invention with Comparative Example 1 in which the iron oxide content was calculated using the fluorescent X-ray analysis method.

Fig. 4 (b) is a graph showing by contrasting Invention Example 1 in which the iron oxide content of the molten slag of which the temperature was 1390 to 1410°C was calculated using the molten slag composition analysis method according to the present invention with Comparative Example 1 in which the iron oxide content was calculated using the fluorescent X-ray analysis method.

Figs. 4 (a) and (b) revealed that regression coefficients $R^2$ calculated from these graphs were respectively 0.9520 and 0.9699. These regression coefficients $R^2$ indicate that the molten slag composition analysis method according to the present invention can estimate the iron oxide content of iron oxide included in the molten slag with extremely high accuracy.

In the molten slag analysis method according to the present invention, the time from the point when the platinum electrodes were immersed into the molten slag until when the estimated values of the basicity and the iron oxide content of the molten slag were derived was within 60 seconds. On the other hand, in the fluorescent X-ray analysis method adopted in Comparative Example 1, the analysis of the slag composition of the molten slag present inside the furnace during converter blowing took ten minutes or longer for sampling of the molten slag and the processing of the obtained sample into a specimen for analysis.

Thus, it was confirmed that the molten slag analysis method according to the present invention was a technique that can quickly and highly accurately analyze the basicity and the iron oxide content of the molten slag.

Industrial Applicability

The molten slag analysis method according to the present invention can quickly and accurately estimate the basicity and the iron oxide content of molten slag without sampling the molten slag during blowing. Thus, this method contributes to development of the related industries, such as the iron manufacturing industry, and is therefore industrially extremely useful.

Reference Signs List

| | |
|---|---|
| 100 | Molten slag analysis device |
| 101 | Crucible |
| 102 | Ramming material |
| 103 | Induction melting furnace |
| 104 | Electrode |
| 104R | Electrode (right side) |
| 104L | Electrode (left side) |
| 105 | Temperature sensor |
| 106 | Potentiostat |
| 107 | Conductive wire |
| 108 | Heat insulation board |
| 200 | Molten slag |

**Claims**

1. A molten slag analysis method that analyzes a constituent of molten slag, comprising:

   a first step of measuring a reference slag temperature of reference molten slag, and measuring a reference impedance of the reference molten slag by applying an alternating current between a pair of electrodes immersed in the reference molten slag at the reference slag temperature;

a second step of creating a constituent content calibration curve from a content of the constituent included in the reference molten slag and the reference impedance measured in the first step;

a third step of measuring a slag temperature of molten-slag-being-measured, and measuring a measured impedance by applying the alternating current between the pair of electrodes immersed in the molten-slag-being-measured at the slag temperature; and

a fourth step of estimating a content of the constituent included in the molten-slag-being-measured based on the measured impedance and the constituent content calibration curve.

2. The molten slag analysis method according to claim 1, wherein

the constituent is iron oxide, and
the constituent content calibration curve is an iron oxide content calibration curve.

3. The molten slag analysis method according to claim 1, wherein:

the constituent is a specific constituent needed to calculate a basicity of the reference molten slag; and
the constituent content calibration curve is a basicity calibration curve.

4. The molten slag analysis method according to any one of claims 1 to 3, wherein
the reference impedance and the measured impedance are measured by applying alternating currents of two selected types of frequencies.

5. The molten slag analysis method according to claim 4, wherein:

a voltage that is applied between the pair of electrodes is 10 mV or higher but 1.0 V or lower; and
the two types of frequencies are respectively 10 Hz or lower and 10 kHz or higher.

6. The molten slag analysis method according to any one of claims 1 to 3, wherein
a difference in immersion potentials in the molten-slag-being-measured between the pair of electrodes is less than 50 mV.

[Fig. 1]

[Fig. 2]

[Fig. 3]

**(a)**

**(b)**

[Fig.4]

**(a)**

**(b)**

# EP 4 737 892 A1

<table>
<tr><td colspan="2"><strong>INTERNATIONAL SEARCH REPORT</strong></td><td>International application No.<br><br><strong>PCT/JP2024/010219</strong></td></tr>
</table>

**A. CLASSIFICATION OF SUBJECT MATTER**

*G01N 27/02*(2006.01)i
FI: G01N27/02 Z

According to International Patent Classification (IPC) or to both national classification and IPC

**B. FIELDS SEARCHED**

Minimum documentation searched (classification system followed by classification symbols)

G01N27/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2022-99837 A (KYUSHU UNIVERSITY) 05 July 2022 (2022-07-05) | 1-6 |
| A | JP 2003-207473 A (HERAEUS ELECTRO NITE KK) 25 July 2003 (2003-07-25) | 1-6 |
| A | KR 10-2023-0021421 A (KYONGGI UNIVERSITY INDUSTRY & ACADEMIA COOPERATION FOUNDATION) 14 February 2023 (2023-02-14) | 1-6 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br><br>**10 May 2024** | Date of mailing of the international search report<br><br>**21 May 2024** |
|---|---|
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br>Telephone No. |

Form PCT/ISA/210 (second sheet) (July 2022)

**INTERNATIONAL SEARCH REPORT**
Information on patent family members

International application No.

**PCT/JP2024/010219**

| Patent document cited in search report | | | Publication date (day/month/year) | Patent family member(s) | Publication date (day/month/year) |
|---|---|---|---|---|---|
| JP | 2022-99837 | A | 05 July 2022 | (Family: none) | |
| JP | 2003-207473 | A | 25 July 2003 | (Family: none) | |
| KR | 10-2023-0021421 | A | 14 February 2023 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7111282 B

- JP 2022110303 A